(19) **Europäisches Patentamt / European Patent Office / Office européen des brevets**

(11) **EP 2 156 851 B1**

(12) **EUROPEAN PATENT SPECIFICATION**

(45) Date of publication and mention of the grant of the patent:
**25.07.2012 Bulletin 2012/30**

(21) Application number: **08764357.3**

(22) Date of filing: **19.05.2008**

(51) Int Cl.:
*A61L 27/06* (2006.01)  *A61L 27/30* (2006.01)

(86) International application number:
**PCT/JP2008/059149**

(87) International publication number:
**WO 2008/143219 (27.11.2008 Gazette 2008/48)**

(54) **METHOD FOR PRODUCTION OF BIOCOMPATIBLE IMPLANT**

VERFAHREN ZUR HERSTELLUNG EINES BIOKOMPATIBLEN IMPLANTATS

PROCÉDÉ D'OBTENTION D'UN IMPLANT BIOCOMPATIBLE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **18.05.2007 JP 2007132708**

(43) Date of publication of application:
**24.02.2010 Bulletin 2010/08**

(73) Proprietor: **National University Corporation Okayama University**
**Okayama-shi, Okayama 700-8530 (JP)**

(72) Inventors:
- **HAYAKAWA, Satoshi**
  **Okayama-shi**
  **Okayama 700-8530 (JP)**
- **OSAKA, Akiyoshi**
  **Okayama-shi**
  **Okayama 700-8530 (JP)**
- **TSURU, Kanji**
  **Fukuoka 8120894 (JP)**
- **SHOZUI, Tetsuya**
  **Mie 5161108 (JP)**

(74) Representative: **Müller-Boré & Partner**
**Patentanwälte**
**Grafinger Straße 2**
**81671 München (DE)**

(56) References cited:
WO-A1-95/13100      WO-A2-2006/043166
JP-A- 2003 235 954     JP-A- 2006 075 500

- **FIRSTOV G S ET AL: "Surface oxidation of NiTi shape memory alloy" BIOMATERIALS, ELSEVIER SCIENCE PUBLISHERS BV., BARKING, GB LNKD- DOI:10.1016/S0142-9612 (02)00244-2, vol. 23, no. 24, 1 December 2002 (2002-12-01), pages 4863-4871, XP004380889 ISSN: 0142-9612**
- **LIU X. ET AL.: 'Light-induced bioactive TiO2 surface' APPLIED PHYSICS LETTERS vol. 88, no. 1, 2006, pages 013905-1 - 013905-3, XP012080436**
- **CHU P.K.: 'Plasma-Treated Biomaterials' IEEE TRANSACTIONS ON PLASMA SCIENCE vol. 35, no. 2, April 2007, pages 181 - 187, XP011176834**
- **WANG X.X. ET AL.: 'A comparative study of in vitro apatite deposition on heat-, H2O2-, and NaOH-treated titanium surfaces' JOURNAL OF BIOMEDICAL MATERIALS RESEARCH vol. 54, no. 2, 2001, pages 172 - 178, XP008124260**

**Description**

The present invention relates to a method for producing a biocompatible implant having a titanium oxide film on the surface of a base material.

In recent years, metal implants have increasingly been used widely in the fields of orthopedics and dentistry, such as artificial bones and artificial tooth roots. For example, when the function of a joint has been lost due to arthrosis deformans or rheumatoid arthritis, medical treatment for regaining the function by exchange to an artificial joint has become general.

As the method for fixing artificial joints to bones, two main types of methods are presently used. One is a technique of filling an adhesive called bone cement into a gap between a bone and an artificial joint to fix them. Since bone cement hardens during the operation, it becomes possible to start rehabilitation early after the operation. However, its use tends to decrease year by year because the risk of causing a shock disease or a blood pressure decline due to excessive compression to the bone marrow during the filling of bone cement has been reported. Another method is a fixing method called cementless fixation, which uses no bone cement. One example is a method of fixing by a mechanical anchoring effect caused by intrusion of a surrounding bone into a porous part formed in the surface of an artificial joint. Since this method can avoid the risk caused by use of bone cement, the cases using the method are increasing rapidly. However, since the time needed for an artificial joint to be fixed to a bone depends on the rate of growth of patient's bone, the patient is required to take a long-period rest and rehabilitation.

In order to shorten the resting period and the rehabilitation period when the aforementioned cementless fixation is adopted, some methods for imparting osteoconductive property to artificial joints have heretofore been investigated. One of them is a method in which osteoconductive property is imparted to the surface of an artificial joint by spraying hydroxyapatite, which is a bone-like component, at high temperatures, and it has already been in practical use. It, however, is supposed that this method has problems that large-scaled equipment for spraying is required, that apatite to be sprayed may be degraded to exposure to high temperature, and that an apatite layer formed may exfoliate.

Patent Document 1 discloses an osteoconductive biomaterial comprising a metal base material containing titanium and a metal oxide layer formed on a surface of the metal base material, wherein at least a surface of the metal oxide layer has a chemical species composed of TiOH. The osteoconductive biomaterial having such a chemical species on its surface is formed by hydrothermally treating, under conditions including a temperature of 100°C or higher and a pressure of 0.1 MPa or higher, a titanium oxide layer obtained by thermally treating a metal base material containing titanium at a temperature of 1000°C or lower. At this time, the preferable thickness of the metal oxide layer formed by the heat treatment is about 3 to 10 $\mu$m. By adopting such a constitution, it is possible to provide a biomaterial with a good osteoconductive property. For example, in Example 1 of Patent Document 1, a sample is disclosed which had been obtained by forming a metal oxide layer of about 5 $\mu$m in a thickness by thermally treating a Ti-29Nb-13Ta-4.6Zr alloy at 800°C for one hour, and hydrothermally treating the resultant under conditions of 120°C and 0.2 MPa while immersing it in a phosphate buffer. And it is disclosed that the sample generated apatite crystals in a simulated body fluid. However, heating the alloy for a long period of time at high temperatures (800°C) inevitably results in lowering the strength of the metal base material. Moreover, when the titanium oxide film is too thick, the film tends to exfoliate easily. On the other hand, Comparative Example 2 of Patent Document 1 discloses that no apatite crystals can be formed by only forming a metal oxide layer without conducting the aforementioned hydrothermal treatment.

Non-Patent Document 1 discloses the result of the observation of apatite forming state by immersing, in a simulated body fluid, a titanium metal flat plate sample on the surface of which an oxide layer had been formed by heat treatment in the air at 400°C for one hour. In the experiment, the container containing the simulated body fluid was a polystyrene container having an upwardly curved bottom surface and a flat-plate sample was immersed therein in such a way that the flat-plate sample was placed on the curved bottom. Then, no apatite was formed on the upper surface of the sample, but formation of apatite only on the under surface (the side which comes into contact with the bottom of a container) was observed. Since the under surface of the sample was in contact with the curved surface of the container, the gap depended on the location, but in general apatite was easily formed at places where there was a gap of about 100 $\mu$m. That is, it is shown that the formation of the apatite is possible only in a restricted environment.

By a so-called sol-gel method, in which a base material such as metal is coated with solution containing an organic titanium compound such as alkoxy titanium, and is subjected to a heat treatment, a titanium oxide film can be formed (see Non-Patent Document 2, for example). It is known that on the surface of the titanium oxide film formed this time, hydroxyapatite is formed in a simulated body fluid. There is a case, however, that no hydroxyapatite is formed by this method when using a certain base material. It is known that when the base material is stainless steel, alumina, or soda-lime glass, for example, no hydroxyapatite is formed on the surface of the titanium oxide film obtained by the sol-gel method (Non-Patent Document 3). This is probably due to that fact that a diffusion component from these base materials to the titanium oxide film inhibits the formation of the hydroxyapatite. For example, the stainless steel is resistant to corrosion, relatively safe to a human body, and easy to be machined. Thus, in utilizing the stainless steel, a surface treatment capable of providing a good osteoconductive property is highly desired.

Non-Patent Document 4 shows results obtained by irradiating a molded article formed by compression-molding titanium oxide powders with light from a mercury lamp, and then, immersing the resultant in a solution having 1.5 times the ion concentration of a simulated body fluid. It has been reported that, as a result, on the surface irradiated with the light, the hydroxyapatite is formed, and on the surface not irradiated with the light, no hydroxyapatite is formed. However, according to this embodiment, even when the solution having 1.5 times the ion concentration of the simulated body fluid is used, no hydroxyapatite is formed in five days, and it took as much as 10 days to form the hydroxyapatite. Thus, it cannot be said that its apatite-forming ability is not be necessarily sufficient.

Non-Patent Document 5 shows results obtained by plasma spray coating titanium oxide powders (30 nm in size) containing 80% of anatase phase and 20% of rutile phase, onto a titanium-alloy base material, irradiating the coated surface with ultraviolet rays, and immersing the resultant into a simulated body fluid. According thereto, it is described that when no ultraviolet rays are irradiated, no apatite is formed, but when the ultraviolet rays are irradiated, the apatite is formed. However, even when the ultraviolet rays are irradiated for 24 hours with a 125-W high-pressure mercury lamp, it took as long as four weeks to form the apatite. Thus, its apatite-forming ability cannot be necessarily sufficient. Moreover, the plasma spray coating requires large-scale equipment and it is difficult to coat the surface of a three-dimensionally shaped article with a uniform film thickness.

Non-Patent Document 6 describes that the surface of a titanium-metal base material is micro-ark oxidized, and then, the resultant surface is irradiated with ultraviolet rays in a simulated body fluid, thereby forming an apatite on the surface. Herein, the micro-ark oxidization is a method in which titanium metal (used as an anode) is immersed into electrolyte, the titanium metal surface is thereby oxidized by applying voltage thereto, and then a titanium oxide film is formed. It is described that in the simulated body fluid, the apatite is formed by irradiating the titanium oxide film containing an anatase crystal with ultraviolet rays from a 1000-W mercury lamp for two hours. However, since the apatite is formed by irradiation of ultraviolet rays in a simulated body fluid, this method is restricted to the usage in which the apatite formed in vitro in advance is implanted. That is, fusion with a bone, along with deposition of the apatite under a human internal environment, is not assumed. In addition, a porous titanium oxide film is formed, and therefore, a surface condition of the shaped base material is changed, and the appearance of the implant is impaired and the metallic luster is lost as well.

Patent Document 2 describes that an osseointegration property is improved by hydroxylating the surface of an implant made of titanium or a titanium alloy, and then, irradiating the surface with ultraviolet rays. Herein, the hydroxylation of the surface of the implant is carried out by etching the implant made of titanium or a titanium alloy with acid, and this means that titanium oxide film is not actively formed. Moreover, the ultraviolet rays are irradiated in order to dissolve and remove organic impurities.

In this context, WO 2006/043166 A2 describes a method for preparing endosseous implants with a high osseointegration degree by means of a titanium dioxide coating thin film in the anatase crystalline form

Patent Document 1: JP-A-2003-235954
Patent Document 2: JP-A-2005-505352
Non-Patent Document 1: Xiao-Xiang Wang et al., "A comparative study of in vitro apatite deposition on heat-, H2O2-, and NaOH-treated titanium", Journal of Biomedical Materials Research, 2001, Vol. 54, p. 172 to 178
Non-Patent Document 2: Toshinobu YOKO et al., "Photoelectrochemical Properties of TiO2 Films Prepared by the Sol-Gel Method", Yogyo-Kyokai-shi, 1987, Vol. 95, p. 150 to 155
Non-Patent Document 3: T. Shozui et al., "In Vitro Apatite-Forming Ability of Titania Films Depends on Their Substrates", Key Engineering Materials, 2007, Vol. 330 to 332, p. 633 to 636, Trans Tech Publications, Switzerland.
Non-Patent Document 4: Toshihiro Kasuga et al., "Apatite formation on TiO2 in simulated body fluid", Journal of Crystal Growth, 2002, p. 235 to 240
Non-Patent Document 5: Xuanyong Liu et al., "Light-induced bioactive TiO2 surface", Applied Physics Letters, 2006, Vol. 88, 013905
Non-Patent Document 6: Yong Han et al., "Photoexcited formation of born apatite-like coatings on micro-arc oxidized titanium", Journal of Biomedical Materials Research, 2004, Vol. 71A, p. 608 to 614

SUMMARY OF INVENTION

TECHNICAL PROBLEM

The present invention has been achieved to solve the aforementioned problems, and an object thereof is to provide a method for producing a biocompatible implant having a titanium oxide film excellent in ability to form hydroxyapatite.

SOLUTION TO PROBLEM

The above-described problems can be solved by providing a method for producing a biocompatible implant, comprising: forming a titanium oxide film containing a rutile-type crystal and having a thickness of 30 to 1500 nm on a surface of the base material made of titanium metal or a titanium alloy by performing a heat treatment on the base material in an oxidizable gas at a temperature of 420 to 790°C to oxidize titanium atoms contained in the base material; and then irradiating the titanium oxide film with ultraviolet rays.
It is preferable that an irradiation amount of ultraviolet rays having a wavelength of 250 to 420 nm be 1 J/cm$^2$ or more. Moreover, it is also preferable that a static contact angle relative to water be five degrees or less.

According to a producing method of the present invention, it is possible to provide a biocompatible implant having a titanium oxide film excellent in ability to form hydroxyapatite.

[FIG. 1]

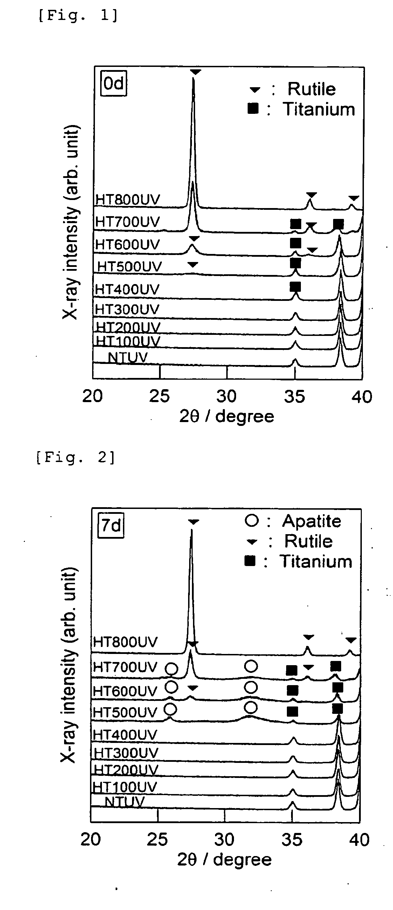

FIG. 1 is a result of a thin film X-ray diffraction measurement of a test piece which has been irradiated with ultraviolet rays and which is to be immersed into a simulated body fluid in Example 1.

[FIG. 2]
FIG. 2 is a result of a thin film X-ray diffraction measurement of a test piece which has been irradiated with ultraviolet rays and which has been immersed into a simulated body fluid for seven days in Example 1.

[FIG. 3]

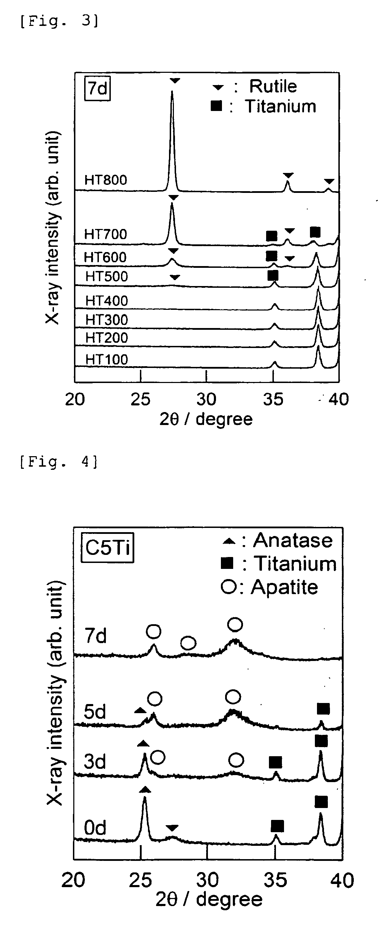

FIG. 3 is a result of a thin film X-ray diffraction measurement of a test piece (not irradiated with ultraviolet rays) which has been immersed into a simulated body fluid for seven days in Example 1.

[FIG. 4]
FIG. 4 is a result of a thin film X-ray diffraction measurement of a test piece ("C5Ti") of which the base material is made of titanium metal and which is not irradiated with ultraviolet rays in Example 2.

[FIG. 5]

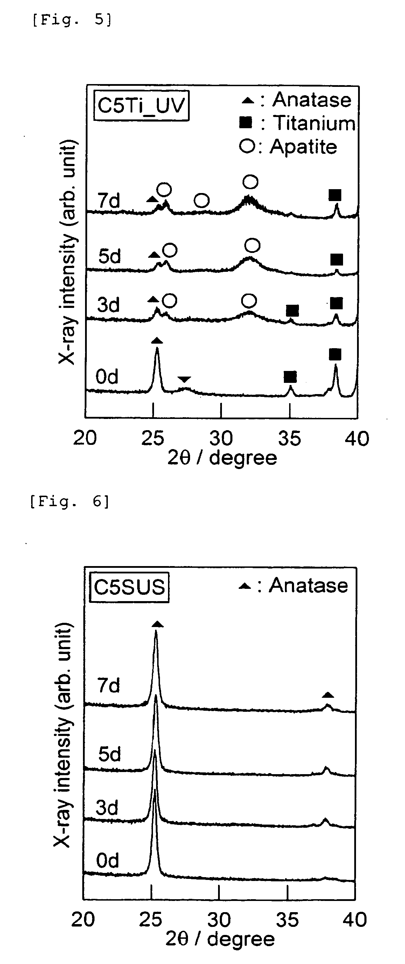

FIG. 5 is a result of a thin film X-ray diffraction measurement of a test piece ("C5Ti_UV")of which the base material is made of titanium metal and which is irradiated with ultraviolet rays in Example 2.

[FIG. 6]
FIG. 6 is a result of a thin film X-ray diffraction measurement of a test piece ("C5SUS") of which the base material is made of stainless steel and which is not irradiated with ultraviolet rays in Example 2.

[FIG. 7]

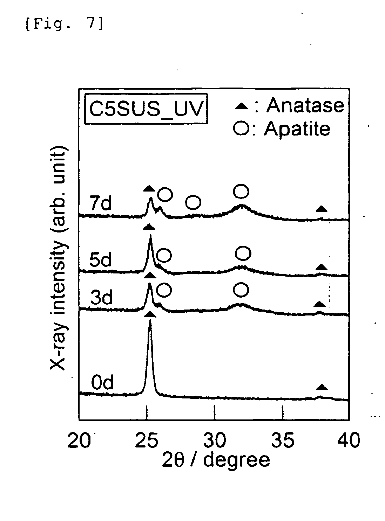

FIG. 7 is a result of a thin film X-ray diffraction measurement of a test piece ("C5SUS_UV") of which the base material is made of stainless steel and which is irradiated with ultraviolet rays in Example 2.

A method for producing a biocompatible implant according to the present invention is a method in which a titanium oxide film is formed on a surface of a base material by performing a heat treatment on the base material, and then, the titanium oxide film is irradiated with ultraviolet rays. Thereby, it becomes easier to form hydroxyapatite on the surface of the titanium oxide film, resulting in the provision of an implant excellent in biocompatibility.

As shown in Non-Patent Documents 4 to 6, it is already known that when the titanium oxide is irradiated with ultraviolet rays, the apatite-forming ability is improved. This is probably due to the fact that the surface of the titanium oxide is photoexcited by the light irradiation, and the resultant surface is thereby in a state that facilitates the formation of the apatite. However, its apatite-forming ability is yet insufficient, and thus, a further improvement is desired. When the present inventors examined, it became obvious that if the titanium oxide film was formed on the surface by thermally treating the base material, the apatite-forming ability caused by the ultraviolet ray irradiation was greatly improved. Reasons for this may probably be explained by an increase in amount of Ti-OH group that is favorable to the formation of an apatite core, for example.

Hereinafter, the present invention will be described in detail.
As described later, when forming a titanium oxide film by oxidizing a base material surface, a base material made of titanium metal or a titanium alloy is used.
A shape of the base material used in the present invention is not particularly restricted, and shaped articles of various shapes according to each application may be used as the base material.

In a producing method of the present invention, it is important that the titanium oxide film be formed on the surface of the base material by thermally treating the base material. When the film of the titanium oxide formed along with the heat treatment is provided, the effect of improving an apatite-forming ability realized by ultraviolet ray irradiation is significant. Moreover, when the titanium oxide film, together with the base material, is thermally treated, a good adhesion of the titanium oxide film to the base material is also provided. In this case, as a method for forming the titanium oxide film by the heat treatment, that in which a metal base material is oxidized so that the oxide film is formed on its surface,

are adopted. Temperatures of the heat treatment are 420 to 790°C. When the heating temperature is less than 250°C, the titanium oxide film is not sufficiently formed, and even when the ultraviolet rays are irradiated, the apatite-forming ability may not be improved. On the other hand, when the heating temperature exceeds 790°C, the crystal may grow too much, making the film fragile, or the crystal structure may be changed to that which is not suitable for the formation of the apatite. More preferably, the temperature of the heat treatment is 750°C or lower, and even more preferably, 650°C or lower.

The thickness of the titanium oxide film formed on the surface of the base material is 30 nm to 1500 nm. When the thickness of the titanium oxide film is less than 30 nm, the apatite-forming ability may be insufficient. When the thickness of the titanium oxide film exceeds 1500 nm, the film may exfoliate.

The method for forming the titanium oxide film is that in which a base material made of titanium metal or a titanium alloy is heated in an oxidizable gas. Firstly, this method will be described below. The base material at this time is made of titanium metal oral or a titanium alloy. When titanium metal is used as the base material, while the resulting implant is excellent in apatite-forming ability, it is occasionally insufficient in strength. That implant therefore is preferably used at regions where a large load is not applied, for example, artificial tooth roots. On the other hand, titanium alloys containing metals other than titanium, some of which may have reduced the apatite-forming ability, are preferably used as an artificial joint, an internal fixation material, an intramedullary nail, etc., at regions which receive so large a load that they are required to have strength because highly strong implants can be obtained therefrom.

The titanium alloy used in the method may be any alloy which contains titanium, and is not particularly restricted. It, however, is preferable that a titanium content be 20% by weight, it is more preferably 50% by weight or more, and it is further preferably 70% by weight or more. Examples of the metal other than titanium to be incorporated in the titanium alloy include aluminum, vanadium, zirconium, tantalum, niobium, palladium and molybdenum. The most common titanium alloy among titanium alloys currently used for medical application, such as Ti-6Al-4V (a titanium alloy containing 6% by weight of aluminum, 4% by weight of vanadium, and titanium as the remainder), may be used. If the content of the metal other than the titanium incorporated in the titanium alloy is less than 0.1% by weight, there is a possibility that the strength may be insufficient in some applications. The content is more preferably 1% by weight or more (at this time, the titanium content is 99% by weight or less), even more preferably 5% by weight or more (the titanium content is 95% by weight or less), and particularly preferably 10% by weight or more (the titanium content is 90% by weight or less). On the other hand, if the content of the metal other than titanium exceeds 50% by weight, the apatite-forming ability may deteriorate. The content is more preferably 40% by weight or less (the titanium content is 60% by weight or more), and it is even more preferably 30% by weight or less (the titanium content is 70% by weight or more).

Because implants having various dimensions or shapes are often needed, the titanium metal or the titanium alloy is shaped into a desired shape in advance. The shaping method is not particularly restricted, and the implants can be shaped by casting, forging, engraving, etc. At this time, at a joining portion with a bone tissue, irregularity may be formed on its surface. According to the method, it is very easy to form the titanium oxide film, with a uniform film thickness, on the surface of a three-dimensionally shaped article.

After the molding in this way, heating is conducted in the oxidizabe gas, and then, the titanium oxide film is formed on the surface. The oxidizable gas used here is not particularly restricted insofar as it is possible to form the titanium oxide film by oxidizing a titanium element on the surface of the base material in that atmosphere. Specifically, it is preferable to heat in an atmosphere containing oxygen, such as in the air. A formation operation of the titanium oxide film according to such a method is very easy. Moreover, the titanium oxide film to be formed is obtained through oxidization of titanium atoms contained in the base material by the heat treatment, and has a good adhesion to the base material of the film. Further, the titanium oxide film to be formed is obtained merely through oxidization of metal atoms contained in the base material, and thus, generally, it provides a high safety for a living body.

In the method, the heating temperature during heating in the oxidizable gas is 420 to 790°C. If the heating temperature is lower than 420°C, an oxide film will be formed insufficiently, and therefore, there is a possibility that the apatite-forming ability may deteriorate. The heating temperature is more preferably 450°C or higher. On the other hand, if the heating temperature exceeds 790°C, there is a possibility that the mechanical strength of the implant may deteriorate due to occurrence of change in the crystal structure of the titanium metal or titanium alloy of the base material. Moreover, the titanium oxide film to be formed may be too thick or the crystal growth may be too excessive, and as a result, there is a possibility that the filmmay become fragile or easily exfoliate. The heating temperature is more preferably 750°C or lower, and even more preferably 650°C or lower. While the heating time is set properly depending upon the relationship with a heating temperature, it is usually from about 1 minute to about 24 hours. The thickness of the titanium oxide film to be formed is as described above, and is 30 nm to 1500 nm. The higher the heating temperature or longer the heating time, the thicker the titanium oxide film to be formed becomes.

The titanium oxide film formed by the method contains a rutile-type crystal. As shown in Examples described later, a thin film X-ray diffraction analysis conducted on the titanium oxide film formed by heat treatment revealed that a diffraction peaks derived from a rutile-type crystal were observed but a diffraction peak arising from an anatase-type crystal was not observed. Therefore, it can be assumed that even if the anatase-type crystal is contained in the titanium oxide film, its amount is small, and when the titanium oxide film is formed through oxidization of a titanium element in the base material, the rutile-type crystal seems to be easily formed directly. Therefore, in the titanium oxide film formed, it is preferable that the diffraction peaks derived from the rutile-type crystal be larger than those derived from the anatase-type crystal, and it is more preferable that in a usual thin film X-ray diffraction measurement, only the diffraction peaks derived from the rutile-type crystal be observed and those derived from the anatase-type crystal be not observed. Conventionally, it is known that it is easier to form the apatite on the anatase-type crystal than on the rutile-type crystal. Given this, the producing method according to the present invention has a greater significant because it provides an excellent apatite-forming ability even in the titanium oxide film containing the rutile-type crystal. Metal elements other than titanium, which are contained in the base material, may be contained in the titanium oxide film to be formed.

An alternative method for forming the titanium oxide film which method is not part of the present invention is that in which the surface of a base material is coated with a liquid containing a titanium compound, and then, the resultant surface is thermally treated, whereby the titanium oxide film is formed according to a sol-gel method. Subsequently, this method will be described below. A base material at this time is not particularly restricted insofar as it is capable of withstanding the heat treatment. Various types of base materials such as metal, glass, and ceramics can be used depending on each application. A shape of the base material used is not particularly restricted, either, and shaped articles of various shapes depending on each application may be used as the base material. According to the alternative method, it is easy to form the titanium oxide film, with a uniform film thickness, on the surface of a three dimensionally shaped article.

Preferably, the base material used in this case is metal in view of machinability or strength. The details of a case where the titanium metal or the titanium alloy is used as metal have already been described above. Other than this, metals such as stainless steel, tantalum, zirconium, nickel, zinc, and cobalt-chromium alloy may be used. According to the alternative method for producing an implant , it is possible to increase the types of base materials on which the apatite can be formed. For example, as is also described in Non-Patent Document 2, it is conventionally known that when the base material on which the titanium oxide film is formed according to a sol-gel method is made of stainless steel, it is difficult to form the apatite. However, even when such a base material is used, if the alternative method is employed, the apatite-forming ability can be improved, and hence, useful. In this case, during the formation of the titanium oxide film by a heat treatment, even when components derived from a base material such as iron atoms are diffused within the film, it is expected that the formation of the apatite is possible. That is, even when the titanium oxide film contains elements other than titanium and oxygen, it is expected that the apatite-forming ability is not easily inhibited. The stainless steel is not only resistant to corrosion and relatively safe to a human body, but also easy to be machined. Therefore, provision of a surface treatment having a good osteoconductive property is highly significant. In this case, the stainless steel is steel containing chromium, and may optionally contain, nickel, manganese, molybdenum, etc. Typical examples may include SUS201, SUS202, SUS301, SUS302, SUS303, SUS304, SUS305, SUS316, and SUS317.

As the base material, glass can also be used, and examples therefor include soda-lime glass, silica glass, borate glass, and titanate glass. As is also described in Non-Patent Document 2, it is conventionally known that when the base material on which the titanium oxide film is formed according to a sol-gel method is made of soda-lime glass, it is difficult to form the apatite. However, even when such a base material is used, if the alternative method is employed, the apatite-forming ability can be improved, and hence, useful. Moreover, as the base material, ceramics can also be used, and examples therefor include alumina, silica, silicon carbide, silicon nitride, and boron nitride. As is also described in Non-Patent Document 2, it is conventionally known that when the base material on which the titanium oxide film is formed according to a sol-gel method is made of alumina, it is difficult to form the apatite. However, even when such a base material is used, if the alternative method is employed, the apatite-forming ability can be improved, and hence, useful.

Liquid to be coated on the surface of the base material in the alternative method is not particularly restricted insofar as it is a liquid containing a titanium compound and is capable of forming the titanium oxide film when a heat treatment is conducted. Sols in which fine titanium oxide particles are dispersed may also be used, and a solution, containing an organic titanium compound, capable of forming a titanium-containing sol after hydrolysis may also be used.

In particular, it is preferable that the coating liquid coated on the base material be a solution or a dispersion containing an organic solvent, water, and at least one selected from the group consisting of titanium alkoxide expressed by the following formula (1), its hydrolysate and its condensation product.

$$R^1{}_n Ti(OR^2)_{4-n} \qquad (1)$$

(in the formula, $R^1$ may be the same or different and is an organic group having a carbon number of 1 to 30, $R^2$ may be the same or different and is an organic group having an alkyl group having a carbon number of 1 to 9, and n is an integer of 0 to 2)

The titanium alkoxide in which n in the formula (1) is 0, i.e., tetra-alkyl ortho-titanate, is preferably used because it provides easy handling. Specifically, preferable examples include tetramethyl orthotitanate, tetraethyl orthotitanate, tetraisopropyl orthotitanate, tetra-n-propyl orthotitanate, and tetra-n-butyl orthotitanate. At this time, it is necessary to contain, in addition to the titanium alkoxide, water for hydrolyzing the titanium alkoxide, and it is also necessary to contain an organic solvent capable of dissolving both the water and the titanium alkoxide.

It is preferable that a water content in terms of 1 mol of titanium alkoxide be 0.2 to 10 mol. More preferably, the water content is 0.5 mol or more, and even more preferably, it is 1 mol or more. On the other hand, more preferably, the water content is 6 mol or less, and even more preferably, it is 4 mol or less. When the water amount is too small, a hydrolysis reaction rate may be too slow, and when the water amount is too large, the hydrolysis reaction may be progressed too rapidly, and as a result, titanium-containing fine particles are agglutinated. However, when the reactivity of the titanium alkoxide is high, coating liquid not containing water can be used where water is absorbed from the surrounding environment, and whereby hydrolysis is conducted.

Preferably, the organic solvent used here is a polar solvent, and examples may include, alcohol, ether, and ketone. Particularly, alcohol is preferable, and preferable examples may include methanol, ethanol, isopropyl alcohol, n-propyl alcohol, and n-butyl alcohol. A content of organic solvent in terms of 1 mol of titanium alkoxide is preferably 5 to 200 mol. More preferably, the content is 10 mol or more, and 100 mol or less. When the content of organic solvent is too small, it is probable that the formed titanium oxide film tends to crack, and as a result, it becomes difficult to form a homogeneous coated film. On the other hand, when the content of organic solvent is too large, the thickness of the coated film to be formed in a single coating operation may be small, and thereby, production efficiency may be decreased.

Moreover, in order to smoothly progress the hydrolysis reaction, it is preferable to contain a hydrolysis catalyst made of acid or alkali. It is preferable that the catalyst be a volatile acid because there is no catalyst residual left in the titanium oxide film to be formed. Preferable examples of acid may include hydrochloric acid, nitric acid, and acetic acid. A content of catalyst is preferably 0.02 to 2 mol in terms of 1 mol of titanium alkoxide. More preferably, the content is 0.05 mol or more, and 1 mol or less.

Moreover, the above-described coating liquid may contain a drying control additive. As a result, the formation of a homogeneous film could be facilitated. As the drying control additive, an organic solvent of which the boiling point is higher than that primarily used in the coating liquid may be used. Examples thereof may include dimethylformamide, dimethylacetamide, and dimethylsulfoxide. Furthermore, the above-described coating liquid may contain a coupling agent. Thereby, the adhesion between the base material and the titanium oxide film could be improved. As the coupling agent, a silane coupling agent, etc., may be used. Metal elements other than the titanium may be contained insofar as not to inhibit the effects of the present invention. However, in viewpoint of the apatite-forming ability, those elements are preferably not contained.

A method for coating the base material with the coating liquid is not particularly restricted. Various coating methods such as dip coating, spray coating, brush coating, and spin coating may be appropriately selected depending on a shape of the base material, etc. Particularly, when coating a base material having a complicated shape, the dip coating is preferable.

After coating with the coating liquid, a heat treatment is conducted. Preferably, the treatment temperature is 250 to 790°C. In a case of a heat treatment at 250°C or lower, the organic substance may remain in the film due to an insufficient resolution of the organic titanium compound, and the strength of the film tobe formedmaybe insufficient. Preferably, the heat treatment is conducted at 350°C or higher, and more preferably, it is conducted at 420°C or higher. On the other hand, when the heating temperature exceeds 790°C, the film may become too fragile due to crystal overgrowth, and the anatase-type crystal may change into a rutile-type crystal, and as a result, the apatite formability may deteriorate. The heating method is not particularly restricted, and a method for heating by using an oven or a heater in the air may be possible.

As described above, the titanium oxide film is formed. In that case, it is preferable to repeat at least two times the operation for the coating and the heat treatment. As a result of multiple coating, a film excellent in uniformity and adhesion can be formed.

Thus, it is preferable that the titanium oxide film formed according to the alternative method contain the anatase-type crystal. The formation of the apatite becomes easier in the anatase-type crystal than in the rutile-type crystal. Therefore, it is preferable that in the titanium oxide film formed, the diffraction peak derive from the anatase-type crystal be larger than that derived from the rutile-type crystal. The titanium oxide film thus formed may contain the metal elements other than the titanium, i.e., a diffusion component from the base material, as described above. The thickness of the titanium oxide film to be formed is as described above, i.e., it is preferably 30 nm or more. The thickness of the titanium oxide film can be adjusted by the titanium compound concentration of the coating liquid or the number of times of coating of the same.

As described above, the titanium oxide film formed by the method of the present invention, the alternative method, and any other similar method is irradiated with ultraviolet rays. The light source is not particularly restricted insofar as it generates ultraviolet rays, and examples there of may include a high-pressure mercury lamp, a xenon lamp, and an LED. With respect to an irradiation amount of ultraviolet rays, the irradiation amount of ultraviolet rays having a wavelength of 250 to 420 nm is preferably 1 $J/cm^2$ or more. More preferably, that amount is 5 $J/cm^2$ or more, even more preferably, the amount is 20 $J/cm^2$ or more, and particularly preferably, the amount is 50 $J/cm^2$ or more. On the other hand, in viewpoint of productivity, generally, the amount is 10000 $J/cm^2$ or less. The wavelength of the ultraviolet rays is more preferably 300 nm or more, and even more preferably, it is 350 nm or more. On the other hand, the wavelength of the ultraviolet rays is more preferably 400 nm or less. In such a preferable wavelength range, the above-described preferable irradiation amounts are preferably satisfied.

When the titanium oxide film is irradiated with the ultraviolet rays, a static contact angle relative to water is decreased. Preferably, the static contact angle of the titanium oxide film obtained after the ultraviolet ray irradiation is five degrees or less. That is, it is preferable the film provide a very hydrophilic surface. On the otherhand, the static contact angle of the titanium oxide film before the ultraviolet ray irradiation is preferably 10 degrees or more, more preferably, it is 15 degrees or more, and even more preferably, it is 20 degrees or more. When the static contact angle was greatly decreased after the ultraviolet ray irradiation compared with that before irradiation, there was a tendency that the formation of the apatite is facilitated. On the other hand, generally, the static contact angle of the titanium oxide film before the ultraviolet ray irradiation is 60 degrees or less.

The implant thus obtained is excellent in apatite-forming ability in the simulated body fluid, and on the surface of the implant, the hydroxyapatite is formed in a relatively shorter period of time, and thus, it is also excellent in bone compatibility. The implant thus obtained can be used widely in orthopedics applications, dental applications, etc., because it is excellent in safety without using no special materials. For example, it can be used preferably in applications such as artificial joints, artificial tooth roots, internal fixation devices and intramedullary nails. It is expected that it can be attached to a bone within a relatively shorter period of time even without using any bone cement.

EXAMPLES

The present invention will be described in more detail with reference to Examples. Each of experiment methods in the Examples is as follows:

(1) Film thickness of the titanium oxide film

Film thicknesses of the titanium oxide film were obtained by observing the cross section of a test piece by using a scanning electron microscope "JSM-6300" (20kV, 300mA) manufactured by Japan Electron Optics Laboratories (JEOL) Ltd. It is noted that it was difficult to observe a test piece that is thermally treated at 400°C or less by using the above-described method. Thus, in consideration of the refractive index of the titanium oxide, an approximate value for the thickness was obtained from its interference color. As a result, it was confirmed that all the test pieces had a thickness of less than 30 nm.

(2) Static contact angle on the surface of the titanium oxide film

An automatic contact angle meter "CA-V" manufactured by Kyowa Interface Science Co., LTD., was used to measure a static contact angle relative to distilled water according to a drop method. One $\mu$l of distilled water was dropped on the surface of a test piece, and after the drop attachment, the static contact angle was automatically measured and calculated. In this case, a $\theta/2$ method was adopted for calculation, the (cross section of) a droplet was assumed to be part of a sphere (circle), and according to the theorem in geometry, a static contact angle $\theta$ was calculated. By using image processing, the diameter (2r) and the height (h) of the droplet were evaluated, and according to the following expressions, $\theta$ was evaluated. In this case, $\theta^*$ denotes an angle formed between: a straight line linking the apex of the droplet and a point with which the droplet surface comes into contact; and a sample substrate.

$$\tan\theta^* = h/r$$

$$\theta = 2 \times \theta^*$$

(3) X-ray diffraction measurement of the titanium oxide film

An X-ray diffractometer "RINT 2000" manufactured by Rigaku Corporation was attached with a thin film attachment (rotating sample board) manufactured by Rigaku Corporation in order to measure the X-ray diffraction. By using the X-ray diffractometer (target CuK$\alpha_1$: 1.5406Å) mounted with a thin film attachment of which the angle of incidence was fixed to one degree, the diffraction was measured under the condition that the output is 40 kV and 200 mA. The measured range in 2$\theta$-angle was 20 to 50 degrees.

Example 1 (formation of the titanium oxide film by heating oxidization)

A metal-titanium test piece (manufactured by Yamamoto Rika: $10 \times 10 \times 2$mm) of which the one surface is mirror-polished was thermally treated at 100 to 800°C for one hour in the air, and thereafter, the resultant test piece was irradiated with ultraviolet rays for one hour. An ultraviolet ray irradiation device used in this case is "HLR100T-2" manufactured by SEN LIGHTS CORPORATION, and is equipped with a high-pressure mercury lamp (lamp power supply: 116V and lamp current: 0.92A). The sample was placed at a position apart by 20 cm from the light source and irradiated. Light intensity at this position was 140 mW/cm$^2$ which was measured with an light intensity meter "25·36-3" manufactured by SEN LIGHTS CORPORATION (this meter has sensibility capable of detection of a wavelength of 365 nm with plus or minus 50 nm). Therefore, a 1-hour exposure dose was 504 J/cm$^2$. The test piece thus irradiated with ultraviolet rays and that not irradiated therewith were immersed into a simulated body fluid at 36.5°C for seven days. The simulated body fluid is a liquid having an inorganic ion concentration substantially equal to that of a human. In its ion concentration: $Na^+$ is 142.0 mM (millimol/liter); $K^+$ is 5.0 mM; $Mg^{2+}$ is 1.5 mM; $Ca^{2+}$ is 2.5 mM; $Cl^-$ is 147.8 mM; $HCO_3^-$ is 4.2 mM; $HPO_4^{2-}$ is 1.0 mM; $SO_4^{2-}$ is 0.5 mM, and pH at 36.5°C is 7.4.

The thicknesses of the titanium oxide film formed by the heat treatment are described in Table 1. In Table 1, "HT800" means a test piece that has undergone a heat treatment at 800°C, for example. The static contact angles relative to water before and after the ultraviolet ray irradiation are described in Table 1. A result of a thin film X-ray diffraction measurement of a test piece (which has been irradiated with the ultraviolet rays and which is not yet immersed into the simulated body fluid) is shown in FIG. 1, a result of the thin film X-ray diffraction measurement of a test piece (which has been irradiated with the ultraviolet rays and which has been immersed into the simulated body fluid for seven days) is shown in FIG. 2, and a result of the thin film x-ray diffraction measurement of a test piece which is not irradiated with the ultraviolet ray and which has been immersed into the simulated body fluid for seven days) is shown in FIG. 3, respectively. In FIGS. 1 to 3, "NT" indicates a test piece not thermally treated, and "UV" indicates a test piece irradiated with the ultraviolet rays, respectively.

As understood from FIG. 2, in the test piece that is thermally treated at 500 to 700°C, and then, irradiated with the ultraviolet rays, the formation of the apatite was confirmed through the thin film X-ray diffraction measurement. When no ultraviolet rays were irradiated as shown in FIG. 3, no apatite was formed irrespective of any heat treatment condition. As understood from FIG. 1, with respect to a test piece thermally treated at 400°C or less, the thin film X-ray diffraction measurement did not confirm the observation of a peak of the rutile-type crystal, and the thickness of the titanium oxide filmwas less than 30 nm. Thus, it can be said that a substantially sufficient titanium oxide film was not formed. As shown in Table 1, even in test pieces thermally treated at 400°C or 300°C, the static contact angle relative to water was greatly decreased after the ultraviolet ray irradiation. Thus, it appears that the quality of the surface of the titanium oxide was improved by irradiation of the ultraviolet ray. However, no apatite was formed on these surfaces. Therefore, it was suggested that the formation of a titanium oxide layer that undergoes a heat treatment at a specific temperature or higher was an essential condition for the formation of the apatite. On the other hand, in the heat treatment at 800°C or higher, the intensity of diffraction peak attributed to the rutile phase was strong. However, no formation of the apatite was confirmed in the simulated body fluid. An excessive crystal growth may prevent the formation of the apatite. However, the details have not been specified. It is acknowledged that there is a tendency that the higher the heat treatment temperature, the smaller the static contact angle relative to water before the ultraviolet ray irradiation, and it is also acknowledged that such a tendency is correlated with a decrease in amount of the apatite to be formed. Therefore, it is important to conduct a heat treatment in an appropriate temperature range, followed by the ultraviolet ray irradiation. Judging from the intensity of the X-ray diffraction peak attributed to the apatite, it is conceivable that the heat treatment at about 500°C is optimal.

Example 2 (formation of the titanium oxide filmby sol-gel method)

(Reference Example)

A metal-titanium test piece of which the one surface is mirror-polished (manufactured by Yamamoto Rika: $10 \times 10 \times 2$ mm) and SUS316L stainless steel of which the one surface is mirror-polished (manufactured by Yamamoto Rika: $10 \times 10 \times 2$ mm) were used as base substrates. Five-minute ultrasonic cleansing operations were conducted in acetone for three times, and the base substrates were cleansed. A sol solution of $Ti(OC_2H_5)_4$: $C_2H_5OH$: $H_2O$: $HNO_3$=1: 50: 2: 0.2 (molar concentration ratio) was prepared, the sol solution was coated on the base substrate with a withdrawal velocity of 6 cm/minute, and the resultant was thereafter dried, followed by heating at 500°C for 10 minutes. This operation was repeated for five times, and as a result, test pieces formed with the titanium oxide film on the surface thereof were obtained. The resultant titanium oxide films were irradiated with the ultraviolet rays, similarly to the Example 1. The test piece thus irradiated with the ultraviolet rays and that not irradiated with the ultraviolet rays were immersed into the simulated body fluid, similarly to the Example 1.

The thicknesses of the titanium oxide film formed by the five coating operations are described in Table 1. In Table 1, the test piece obtained by coating a titanium-metal base substrate for five times is written as "C5Ti", and the test piece obtained by coating a stainless-steel base substrate for five times is written as "C5SUS", respectively. The static contact angles relative to water before and after the ultraviolet ray irradiation are described in Table 1. A result of a thin film X-ray diffraction measurement of a test piece ("C5Ti") of which the base material is titanium metal and which is not irradiated with the ultraviolet rays is shown in FIG. 4; a result of a thin film X-ray diffraction measurement of a test piece ("C5Ti_UV") of which the base material is titanium metal and which is irradiated with the ultraviolet rays is shown in FIG. 5; a result of a thin film X-ray diffraction measurement of a test piece ("C5SUS") of which the base material is stainless steel and which is not irradiated with the ultraviolet rays is shown in FIG. 6; and a result of a thin film X-ray diffraction measurement of a test piece ("C5SUS_UV") of which the base material is stainless steel and which is irradiated with the ultraviolet rays is shown in FIG. 7, respectively. In FIGS. 4 to 7, "0d" indicates a test piece before the immersion into the simulated body fluid, and "3d", "5d", and "7d" indicate test pieces immersed into the simulated body fluid for three, five, and seven days, respectively.

As understood from the results of the thin film X-ray diffraction measurement of the test pieces before immersion into the simulated body fluid in FIGS. 4 to 7, it is seen that the titanium oxide film formed on the base material contains the anatase-type crystal. When the base material is titanium metal, the apatite is formed either when not irradiated with the ultraviolet rays (FIG. 4) or when irradiated with the ultraviolet rays (FIG. 5). On the other hand, when the base material is stainless steel, it is seen that when no ultraviolet rays are irradiated, no apatite is formed (FIG. 6), and when the ultraviolet rays are irradiated, the apatite is formed (FIG. 7). When using the titanium oxide film formed by the sol-gel method (it should be noted that the apatite-forming ability differs depending on types of base material), the formation of the apatite is enabled even when a base material on which it has been believed to be difficult to form the apatite is used. This achieves a wider selection of base materials.

[Table 1]

| Test piece | Thickness of titanium oxide films | | Static contact angle relative to water | |
|---|---|---|---|---|
| | Film thickness | Standard deviation | Before UV irradiation | After UV irradiation |
| | (nm) | (nm) | (degree) | (degree) |
| HT800 | 1739 | 265 | $9.2 \pm 0.3$ | 2.3 |
| HT700 | 897 | 245 | $16.7 \pm 1.3$ | 4.1 |
| HT600 | 224 | 69 | $21.8 \pm 3.0$ | 0.7 |
| HT500 | 117 | 15 | $34.5 \pm 1.7$ | 0.4 |
| HT400 | <30 | - | $1.3 \pm 4.9$ | 1.7 |
| HT300 | - | - | $1.6 \pm 0.9$ | 4.3 |
| C5Ti | 303 | 11 | $26.3 \pm 2.0$ | 3.1 |
| C5SUS | 294 | 10 | $38.6 \pm 1.7$ | 2.9 |

**Claims**

1. A method for producing a biocompatible implant, comprising:

    forming a titanium oxide film containing a rutile-type crystal and having a thickness of 30 to 1500 nm on a surface of the base material made of titanium metal or a titanium alloy by performing a heat treatment on the base material in an oxidizable gas at a temperature of 420 to 790°C to oxidize titanium atoms contained in the base material; and
    then irradiating the titanium oxide film with ultraviolet rays.

2. The method for producing a biocompatible implant according to claim 1, wherein an irradiation amount of ultraviolet rays having a wavelength of 250 to 420 nm is 1 $J/cm^2$ or more.

**Patentansprüche**

1. Verfahren zur Herstellung eines biokompatiblen Implantats, umfassend:

   das Bilden eines Titanoxidfilms, enthaltend einen Kristall vom Rutil-Typ, und
   eine Dicke von 30 bis 1500 nm aufweisend, auf einer Oberfläche des Basismaterials, hergestellt aus Titanmetall oder einer Titanlegierung, durch Durchführen einer Wärmebehandlung auf dem Basismaterial in einem oxidierbaren Gas bei einer Temperatur von 420 bis 790°C, um Titanatome, enthalten in dem Basismaterial, zu oxidieren, und
   anschließend das Belichten des Titanoxidfilms mit ultravioletten Strahlen.

2. Verfahren zur Herstellung eines biokompatiblen Implantats gemäß Anspruch 1, wobei eine Belichtungsmenge an ultravioletten Strahlen mit einer Wellenlänge von 250 bis 420 nm 1 J/cm$^2$ oder mehr beträgt.

**Revendications**

1. Procédé de production d'un implant biocompatible comprenant :

   la formation d'un film d'oxyde de titane contenant un cristal de type rutile et ayant une épaisseur de 30 à 1500 nm sur une surface du matériau de base réalisée en métal titane ou en un alliage de titane, en effectuant un traitement thermique sur le matériau de base, dans un gaz oxydable, à une température de 420 à 790°C, afin d'oxyder les atomes de titane contenus dans le matériau de base ; et
   ensuite l'irradiation du film d'oxyde de titane avec des rayons ultraviolets.

2. Procédé de production d'un implant biocompatible selon la revendication 1, dans lequel une quantité d'irradiation de rayons ultraviolets, ayant une longueur d'onde de 250 à 420 nm, est de 1 J/cm$^2$ ou plus.

[Fig. 1]

[Fig. 2]

[Fig. 3]

[Fig. 4]

[Fig. 5]

[Fig. 6]

[Fig. 7]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006043166 A2
- JP 2003235954 A
- JP 2005505352 A

### Non-patent literature cited in the description

- **Xiao-Xiang Wang et al.** A comparative study of in vitro apatite deposition on heat-, H2O2-, and NaOH-treated titanium. *Journal of Biomedical Materials Research,* 2001, vol. 54, 172-178
- **Toshinobu YOKO et al.** Photoelectrochemical Properties of TiO2 Films Prepared by the Sol-Gel Method. *Yogyo-Kyokai-shi,* 1987, vol. 95, 150-155
- In Vitro Apatite-Forming Ability of Titania Films Depends on Their Substrates. **T. Shozui et al.** Key Engineering Materials. Trans Tech Publications, 2007, vol. 330-332, 633-636
- **Toshihiro Kasuga et al.** Apatite formation on TiO2 in simulated body fluid. *Journal of Crystal Growth,* 2002, 235-240
- **Xuanyong Liu et al.** Light-induced bioactive TiO2 surface. *Applied Physics Letters,* 2006, vol. 88, 013905
- **Yong Han et al.** Photoexcited formation of born apatite-like coatings on micro-arc oxidized titanium. *Journal of Biomedical Materials Research,* 2004, vol. 71A, 608-614